# EUROPEAN PATENT APPLICATION

(11) **EP 0 801 950 A1**
(43) Date of publication of application: **22.10.1997**
(21) Application number: 97105094.3
(22) Date of filing: 26.03.1997
(51) Int. Cl.: A61K 31/34, A61K 9/20, A61K 9/48, A61K 47/18

(54) **Pharmaceutical composition for diuretic and antihypertensive oral therapy comprising furosemide and tromethamine**

(30) Priority: 17.04.1996 IT MI960736
(71) Applicant: RICERCHE DI SCHIENA S.N.C. DEL DR. MICHELE G. DI SCHIENA & C., I-20080 Cisliano (Milano) (IT); Lifepharma s.r.l., 20099 Sesto san Giovanni (MI) (IT)
(72) Inventor: Di Schiena, Michele Giuseppe, 20080 Cisliano, MI (IT)
(74) Representative: Frignoli, Luigi

(57) **Abstract**

A pharmaceutical composition comprising Furosemide in association with Tromethamine usable for achieving a rapid therapeutic response in diuretic and antihypertensive therapy.

## Description

This invention relates to pharmaceutical compositions for use in the therapeutic treatment of water retention and hypertension in the human and veterinary field.

A compound widely used for its effectiveness and safety in diuretic and antihypertensive therapy is 4-chloro-N-furfuryl-5-sulphamoylanthranilic acid, commonly known as Furosemide. This compound is fully described, for example, in "MARTINDALE, The Extra Pharmacoepia, 27th Ed."

When Furosemide is administered orally it has however the drawback of manifesting its therapeutic action a rather lengthy time after its administration, ie a time of between 1 and 4 hours after administration, as also stated on page 566 of the said "MARTINDALE, The Extra Pharmacoepia, 27th Ed.".

The time required for manifestation of the therapeutic action is determined by monitoring the diuresis: reducing this time (which in the case of oral administration of Furosemide is between 1 and 4 hours, as already stated) would be of considerable practical importance because it would enable - as required by the most modern therapeutic practice - the pathological manifestations (sometimes very serious) to be dominated in a shorter time and which in this specific case are related to water retention.

It has been sought to accelerate the manifestation of the therapeutic action of Furosemide by associating with it a large number of compounds, including those commonly used in the pharmaceutical field, such as lecithin, cholic acids, saccharose esters, amino acids, amines, alcohols, acids, etc., but in no case was the desired advantage appreciably obtained.

It has been surprisingly found that by orally administering Furosemide in association with Tromethamine, the time for the therapeutic response (due to the Furosemide) determined by monitoring the diuresis, is on an average about 15 minutes, ie considerably less than that achieved without the aforesaid association. This result is extremely important, as is immediately apparent.

Tromethamine, ie 2-amino-2-hydroxymethyl-1,3-propanediol, is a well known compound, also known as Trometamol or THAM.

It has been found that with the compositions of the present invention, the known therapeutic properties and the low toxicity of Furosemide remain unaltered.

The compositions comprising Furosemide in association with Tromethamine can be formulated in all ways known in the pharmaceutical art for oral administration, as tablets, capsules (including so-called soft capsules), chewing gum, syrups, perioral absorption forms, single-dose sachets, caramels etc. The dosages for patient administration are comparable with those scheduled for the administration of equal quantities of Furosemide.

The association of the present invention can also be administered parenterally, but in this case without particular therapeutic advantages being obtained.

It has been found that in the compositions of the invention, between 0.3 g and 0.4 g of Tromethamine, and preferably between 0.35 and 0.39 g of Tromethamine, can be associated with 1 g of Furosemide. Depending on the pharmaceutical forms to be obtained, normal pharmaceutical excipients can be added.

Other active principles with complementary therapeutic activity can be added to the Furosemide-Tromethamine association, such as: cardiac glycosides, antiarrhythmics, cardiac stimulators, vasodilators, prostaglandins, antiadrenergics with central action, ganglioblockers with peripheral action, substances acting on the arteriolar smooth musculature and on the renin-angiotensin system, alkaloids, thiazides, sulphonamides, potassium salts, aryloxyacetic acid derivatives, pyrazolones, aldosterone antagonists, beta blockers, calcium antagonists, enzymes, anticoagulants, anti-anxiety agents, sedatives (including vegetable) etc.

To clarify the method of preparation of the compositions of the present invention, some non-limiting illustrative examples are given hereinafter:

### EXAMPLE 1

1000 g of Furosemide and 350 g of Tromethamine are carefully mixed together. The resultant powder is then passed through a 45 mesh sieve. The mixture is used to prepare the various formulations for oral use.

### EXAMPLE 2

The procedure of Example 1 is followed, but using 390 g of Tromethamine.

### EXAMPLE 3

The mixture of Example 2 is divided into container caps such that each cap contains 600 mg of Furosemide.

The container cap is supported on a bottle containing 10 ml of a solution formed from: ethanol 5% - acesulfame 0.1% - sterile distilled water to make up to 100%. At the moment of use the contents of the container cap are mixed with the solution.

The resultant mixture is suitable for administration in droplet form.

## Claims

1. A pharmaceutical composition for diuretic and antihypertensive oral therapy, characterised by comprising Furosemide in association with Tromethamine in a ratio of 0.3-0.4 g of Tromethamine per 1 g of Furosemide.

2. A pharmaceutical composition as claimed in claim 1, characterised by comprising between 0.35 g and 0.39 g of Tromethamine per 1 g of Furosemide.
